# EUROPÄISCHE PATENTSCHRIFT

(11) **EP 1 765 900 B1**
(45) Veröffentlichungstag und Bekanntmachung des Hinweises auf die Patenterteilung: **06.10.2010**
(21) Anmeldenummer: 05746591.6
(22) Anmeldetag: 22.06.2005
(51) Int. Cl.: C08G 18/28, C08G 18/48, C09D 7/00

(54) **VERDICKUNGSMITTEL AUF POLYURETHANBASIS**
THICKENING AGENT BASED ON POLYURETHANE
AGENTS EPAISSISSANTS A BASE DE POLYURETHANE

(30) Priorität: 01.07.2004 DE 102004031786
(43) Veröffentlichungstag der Anmeldung: 28.03.2007
(73) Patentinhaber: Cognis IP Management GmbH, 40589 Düsseldorf (DE)
(72) Erfinder: SCHIEFERSTEIN, Ludwig, 40882 Ratingen (DE); PIETSCH, Oliver, 45481 Mülheim (DE)
(86) Internationale Anmeldenummer: PCT/EP2005/006743
(87) Internationale Veröffentlichungsnummer: WO 2006/002813

(56) Entgegenhaltungen:
- WO-A-93/10166
- US-A- 4 079 028

## Beschreibung

### Gebiet der Erfindung

Die Erfindung betrifft Verdickungsmittel basierend auf einer wäßrigen Zubereitung nichtionischer in Wasser dispergierbarer oder löslicher Polyurethane spezieller Struktur.

### Stand der Technik

Polyurethanlösungen oder -dispersionen in wasserverdünnbarer wäßriger oder überwiegend wäßriger Phase werden vom Fachmann als HEUR-Verdicker bezeichnet (das Akronym HEUR leitet sich von "nonionic hydrophobically modified ethylene oxide urethane block copolymer" ab) und bereits seit längerem in den unterschiedlichsten Anwendungsfeldern zur Verdickung wasserbasierter Dispersionsfarben eingesetzt.

Die bereits Ende der 70er Jahre in US-A-4,079,028 beschriebenen Verdickungsmittel vom HEUR-Typ sind aufgebaut aus linearen und/oder verzweigten Polyethylenglycolblöcken und Hydrophobsegmenten, die in der Regel über Urethangruppen (bei Verwendung von Aminen statt Alkoholen resultieren Harnstoffgruppen) miteinander verknüpft sind.

Als Wirkprinzip für die verdickende Wirkung der HEUR-Verdicker wird angenommen, dass die Polyethylenglykolsegmente die Wasserverträglichkeit sicherstellen und die Hydrophobsegmente über eine Assoziation untereinander sowie mit dispergierten Bindemittelteilchen der zu verdickenden Dispersionsfarbe in dieser einen viskositätsgebenden dreidimensionalen Molekülverbund aufbauen.

Bevorzugte hydrophobe Bausteine in marktüblichen HEUR-Verdickem sind längerkettige, in der Regel monofunktionelle Alkohole, wie beispielsweise n-Octanol, n-Dodecanol, iso-Tridecylalkohol, iso-Nonylphenol oder Ricinolsäuremethylester. Diese Alkohole kommen überwiegend als solche, aber auch in Form ihrer Additionsprodukte mit einigen wenigen Äquivalenten Ethylenoxid zum Einsatz.

Die in marktüblichen HEUR-Verdickern überwiegend verwendeten mehrfunktionellen Isocyanat-Bausteine sind in der Regel difunktionell. Beispielsweise kommen Methylenbis-(4-cyclohexyl)diisoyanat, m/p-Tetramethylenxylylendiisocyanat, Hexamethylendiisocyanat, 2,4-Toluylendisocyanat, Trimethylhexamethylendiisocyanat oder 4/2,4'-Diphenylmethandiisocyanat zum Einsatz. Die in marktüblichen HEUR-Verdickem verwendeten Polyethylenglycol-Bausteine sind in der Regel ebenfalls difunktionell und weisen Molekulargewichte im Bereich einiger Tausend Dalton, beispielsweise 4500 oder 10000 Dalton, auf.

Die Einsatzverhältnisse der einzelnen Bausteine von HEUR-Verdickem - seien es nun verzweigte oder unverzweigte Polyethylenglykole, mono- oder mehrfunktionelle Hydrophobalkohole, vorethoxylierte mono- oder mehrfunktionelle Hydrophobalkohole, kettenverlängernd eingesetzte di- oder mehrfunktionelle kurzkettige Alkohole - werden in der Regel so gewählt, dass jedem noch über eine Hydroxylgruppe reaktiven Ethylenglykolsegmentende jeweils ein Hydrophobalkohol zur Verfügung steht.

Die hydroxylterminierten Synthesebausteine von HEUR-Verdickern werden durch Reaktion mit di- oder mehrfunktionellen Isocyanaten miteinander verbunden, wobei die Equivalenteinsatzverhältnisse der miteinender zur Additionsreaktion zu bringenden Isocyanat- sowie der "H-aciden" Gruppen (in der Regel OH-Gruppen; es sind aber auch NH₂-Gruppen möglich) so gewählt werden, dass jedem "H-aciden"Gruppenäquivalent, also in der Regel jeder OH-Gruppe mindestens geringfügig weniger als ein Isocyanatgruppenäquivalent gegenüber steht. Mit anderen Worten: Das Äquivalentverhältnis OH : NCO wird in der Regel auf einen Wert von mindestens 1 : 1 eingestellt, wobei idealerweise 1 : 1 angestrebt wird oder die OH-Gruppen im Vergleich zu den NCO-Gruppen um 5-10% überwiegen (was einem Äquivalentverhältnis von OH : NCO im Bereich von 1,05 : 1 bis 1,1 : 1 entspricht), um sicherzustellen, daß das Endprodukt (der HEUR-Verdicker) keine freien NCO-Gruppen enthält, die unerwünscht sind, einerseits aus toxikologischen Gründen, andererseits weil sie beim späteren Einsatz in zu verdickenden Rezepturen unerwünschte Folgereaktionen mit Rezepturbestandteilen eingehen können. Dieses Grundprinzip, daß nämlich bei der Herstellung von HEUR-Verdickem die OH-Gruppen der Polyethylenglykol- und Hydrophobalkohol-Bausteine im Vergleich zu den NCO-Gruppen der Isocyanat-Bausteine mit ca. 5-10% leicht überwiegen,ist auch bereits Bestandteil der Lehre der oben genannten US-A-4,079,028 (vergleiche dort Spalte 3, Zeilen 17 ff).

Neuerdings gewinnen HEUR-Verdicker zunehmendes Interesse als Verdickungsmittel für disperse kosmetische Zubereitungen (vergl. z.B. EP-A-787,486).

### Beschreibung der Erfindung

Aufgabe der Erfindung war es, Verdickungsmittel auf Polyurethanbasis bereitzustellen, die gegenüber den Polyurethanverdickungsmitteln aus dem Stand der Technik bei vergleichsweise geringerer Eigenviskosität der Verdickungsmittel in ihrer Konfektionierungsform bei gleicher Menge eine erhöhte Viskosität des verdickten Produkts erreicht werden. Außerdem sollten die Verdickungsmittel gewünschtenfalls ohne die Verwendung von flüchtigen organischen Lösungsmitteln herstellbar sein.

Es wurde nun überraschenderweise gefunden, dass die Verdickungswirksamkeit von HEUR-Verdickungsmitteln deutlich gesteigert werden kann, wenn
1. das Verhältnis von (gegenüber NCO-Gruppen) reaktiven funktionellen Gruppen mit mindestens einem Zerewitinoff-aktiven Wasserstoffatom (ZH-Gruppen) pro Molekül einer hydrophoben Verbindung (bei Alkoholen wären dies OH-Gruppen) zu reaktiven OH-Gruppen eines hydrophilen Polyols deutlich über den üblichen Wert von eins hinaus angehoben wird und
2. der Summe von OH- und ZH-Gruppen deutlich mehr als ein Äquivalent mehrfunktioneller Isocyanate zur Verfügung gestellt wird.
   Gegenstand der vorliegenden Erfindung sind Verdickungsmittel, die auf einer wäßrigen Zubereitung nichtionischer, in Wasser dispergierbarer oder in Wasser löslicher Polyurethane basieren, wobei diese Polyurethane herstellbar sind durch Umsetzung von

(a) ein oder mehreren hydrophilen Polyolen (a), die pro Molekül mindestens zwei OH-Gruppen und mindestens zwei funktionelle Gruppen, die ausgewählt sind aus den Funktionen -O- (Ethergruppen) und -COO- (Estergruppen), wobei das Molgewicht dieser hydrophilen Verbindungen mindestens 300 beträgt, enthalten,
(b) ein oder mehreren hydrophoben Verbindungen mit mindestens einem Zerewitinoff-aktiven Wasserstoffatom pro Molekül, wobei das Molgewicht dieser hydrophoben Verbindungen im Bereich von 100 bis 500 liegt und wobei pro Molekül dieser hydrophoben Verbindungen mindestens eine lineare oder verzweigte, gesättigte oder ungesättigte Alkylkette mit mindestens fünf aufeinander folgenden C-Atomen enthalten ist, die nicht mit Heteroatomen verknüpft ist, und
(c) ein oder mehreren, mindestens difunktionellen Isocyanaten,
dadurch gekennzeichnet, daß die Verbindungen a), b) und c) in den Äqivalentverhältnissen OHₐ): ZH_{b}): NCO_{c}) von 1 : (1 +x) : 2(1 +y) miteinander umgesetzt werden, mit den Maßgaben, daß folgende Bedingungen gelten:
- x ist eine Zahl im Bereich von 0,05 bis 1,2,
- y ist eine Zahl im Bereich von (0,2 bis 1,05) und
- Äquivalent verhältnis NCO_{c)} > (OHₐ₎ + ZH_{b)}).

Der Ausdruck OHₐ) bezeichnet die primären (terminalen) OH-Gruppen der Verbindungen a). Der Ausdruck ZH_{b}) bezeichnet die gegenüber NCO-Gruppen reaktiven funktionellen Gruppen mit Zerewitinoff-aktivem Wasserstoffatom b). Der Ausdruck NCO_{c)} bezeichnet die Isocyanatgruppen der Verbindungen c).

Im Rahmen der vorliegenden Erfindung handelt es sich bei den Äquivalenten der Verbindungen a) um OH-Äquivalente, bei den Verbindungen b) um Zerewitinoff-aktive-Wasserstoff-Äquivalente und bei den Verbindungen c) um NCO-Äquivalente.

Obwohl der Begriff des Äquivalents dem Fachmann auf dem hier in Rede stehenden Gebiet der Polyurethanchemie geläufig ist, sei nachstehend der Klarheit halber dargestellt, was darunter zu verstehen ist.

Der Ausdruck Äquivalente ist im üblichen Sinne zu verstehen und fokussiert auf die zur Verfügung stehenden reaktiven Gruppen von Molekülen. So enthält beispielsweise 1 mol eines Monoalkohols 1 mol an OH-Gruppen; 1 mol eines Diols enthält 2 mol an OH-Gruppen, 1 Mol eines Triols enthält drei mol an OH-Gruppen, usw. Ganz analog enthält 1 mol eines Diisocyanates (NCO-Funktionalität = 2) 2 mol an NCO-Gruppen, ein mol eines Polyisocyanatgemisches mit einer (mittleren) Funktionalität von 2,3 enthält durchschnittlich 2,3 mol an NCO-Gruppen, usw. Will man beispielsweise Alkohole und Isocyanate miteinander derart umsetzen, daß die eingesetzten Verbindungen bezogen auf die OH- bzw. NCO-Gruppen in bestimmten Verhältnissen stehen sollen, so empfiehlt es sich, anstelle von Gewichts- oder Molverhältnissen auf die Verhältnisse der reaktiven Gruppen abzustellen. Dieses OH : NCO-Verhältnis bezeichnet man als das Äquivalentverhältnis.

Allgemein ausgerückt ist das Äquivalentverhältnis das Zahlenverhältnis definierter reaktiver Gruppen in den eingesetzten Reaktanden.

Der Anschaulichkeit halber sei zusätzlich durch ein praktisches Beispiel erläutert, wie man ein Äquivalentverhältnis auf einfache Weise ermittelt. Setzt man beispielsweise im Sinne der erfindungsgemäßen Lehre
- 1 mol eines Polyethylenglykols (PEG, OH-Funktionalität =2) mit zwei OH-Gruppen pro Molekül mit
- 4 mol eines Hydrophobalkohols (OH-Funktionalität =1) mit einer OH-Gruppe pro Molekül und
- 4 mol eines Diisocyanates (NCO-Funktionalität =2)
zum Polyurethan um, dann enthält
- das eingesetzte PEG 2 mol OH-Gruppen,
- der eingesetzte Hydrophobalkohol 4 mol OH-Gruppen und
- das eingesetzte Diisocyanat 8 mol NCO-Gruppen.

Das Zahlenverhältnis der OH-Gruppen des Polyethylenglykols zu den OH-Gruppen des Hydrophobalkohols zu den NCO-Gruppen des Diisocyanats beträgt mithin 2 : 4 : 8 oder 1 : 2 : 4. Oder umgekehrt: Will man beispielsweise die gerade genannten Komponenten (PEG, Hydrophobalkohol und Diisocyanat) in einem Äquivalentverhältnis von 1 : 3 : 3 umsetzen, dann hat man Polyethylenglykol, Hydrophobalkohol und Diisocyanat in einem Molverhältnis von 0,5 : 3 : 1,5 oder 1 : 6 : 3 einzusetzen.

Der Klarheit und Eindeutigkeit halber sei ausdrücklich festgestellt, daß sich y durch Multiplikation ergibt. Der für y angegebene Ausdruck, nämlich "(0,2 bis 1,05) x" bedeutet mithin, daß x - wofür jeweils eine konkrete Zahl aus dem für x angegebenen Bereich einzusetzen ist - mit einer Zahl aus dem Bereich 0,2 bis 1,05 zu multiplizieren ist.

In einer Ausführungsform setzt man die Verbindungen a), b) und c) in den Äqivalentverhältnissen OHₐ₎ : ZH_{b)} : NCO_{c)} von 1 : (1 +x) : 2 (1 +y) miteinander um, wobei x eine Zahl im Bereich von 0,05 bis 1,2 und y eine Zahl im Bereich von (0,2 bis 1,05) x ist.

In einer weiteren Ausführungsform setzt man die Verbindungen a), b) und c) in den Äqivalentverhältnissen OHₐ₎ : ZH_{b)} : NCO_{c)} von 1 : (1 +x) : 2 (1 +y) miteinander um, wobei x eine Zahl im Bereich von 0,15 bis 1,1 und y eine Zahl im Bereich von (0,5 bis 1,03) x ist.

In einer bevorzugten Ausführungsform setzt man die Verbindungen a), b) und c) in den Äqivalentverhältnissen Ohrₐ₎ . ZH_{b)} : NCO_{c)} von 1 : (1 +x) : 2 (1 +y) miteinander um, wobei x eine Zahl im Bereich von 0,3 bis 1,05 und y eine Zahl im Bereich von (0,5 bis 1,02) x ist.

In einer besonders bevorzugten Ausführungsform setzt man die Verbindungen a), b) und c) in den Äqivalentverhältnissen OHₐ₎ : ZH_{b)} : NCO_{c)} von 1 : (1 +x) : 2 (1 +y) miteinander um, wobei x eine Zahl im Bereich von 0,5 bis 1,02 und y eine Zahl im Bereich von (0,7 bis 1,01) x ist.

### Zu den Komponenten (a)

Die hydrophilen Polyole (a) enthalten per definitionem pro Molekül mindestens zwei OH-Gruppen und mindestens zwei funktionelle Gruppen, die ausgewählt sind aus den Funktionen -O- (Ethergruppen) und -COO- (Estergruppen), wobei das Molgewicht dieser hydrophilen Verbindungen mindestens 300 und vorzugsweise mindestens 1000 beträgt. Bei der Komponente (a) handelt es sich mithin um den hydrophilen Molekülbaustein der erfindungsgemäßen Polyurethane vom Typ HEUR. Ausdrücklich sei darauf hingewiesen, daß sich die Verbindungen (a) grundsätzlich von denjenigen Verbindungen (b) unterscheiden, die nicht hydrophil, sondern hydrophob sind.

Als Verbindungen (a) sind beispielsweise die Polymerisationprodukte des Ethylenoxids, deren Misch- oder Pfropfpolymerisationsprodukte sowie die durch Kondensation von mehrwertigen Alkoholen oder Mischung derselben und die durch Ethoxylierung von mehrwertigen Alkoholen, Amiden, Polyamiden und Aminoalkoholen gewonnenen Polyether geeignet. Beispiele geeigneter Verbindungen (a) sind etwa Polyethylenglykole, Anlagerungsprodukte von Ethylenoxid an Trimethylolpropan, EO-PO-Blockcopolymere, OH-terminierte Polyester wie beispielsweise solche vom Typ der mehrfunktionellen Polycaprolactone.

Bevorzugte Verbindungen (a) sind Polyetherpolyole. Dies sind solche hydrophilen Polyole (a), die pro Molekül mindestens zwei OH-Gruppen und mindestens zwei Funktionen -O-(Ethergruppen) enthalten. Diese Polyetherpolyole sind in aller Regel so stark hydrophil, daß sie bei Raumtemperatur (20 °C) wasserlöslich sind.

Zur Herstellung der erfindungsgemäßen Polyurethane eignen sich bevorzugt solche Polyetherpolyole, die zumindestens überwiegend Polyethylenglykol enthalten. Besonders gute Ergebnisse werden erzielt, wenn diese Polyethenglykole einen mittleren Gehalt an Alkoxyeinheiten im Bereich von 20 bis 400 aufweisen.

Bevorzugt sind als Verbindungen (a) Diole der allgemeinen Formel HO-(CH₂-CH₂-O)ₙ-H, wobei n die Werte 20 bis 400 annehmen kann. Hierbei handelt es sich um Polyethylenglykole, die Kondensationsprodukte des Ethylenoxids mit Ethylenglykol oder Wasser darstellen. Vorzugsweise stellt man das Molgewicht dieser Polyethylenglykole auf Werte im Bereich von 1.000 bis 15.000 ein.

### Zur Komponente (b)

Bei Komponente (b) handelt es sich um hydrophobe Verbindungen mit mindestens einem Zerewitinoff-aktiven Wasserstoffatom pro Molekül, wobei das Molgewicht dieser hydrophoben Verbindungen im Bereich von 100 bis 500 liegt und wobei pro Molekül dieser hydrophoben Verbindungen mindestens eine lineare oder verzweigte, gesättigte oder ungesättigte Alkylkette mit mindestens fünf aufeinander folgenden C-Atomen enthalten ist, die nicht mit Heteroatomen verknüpft ist. Der Begriff "Heteroatome" ist dem Fachmann bekannt, Kohlenstoff und Wasserstoff sind selbstverständlich keine Heteroatome.

Bekanntlich bezeichnet man an N, O oder S gebundenen Wasserstoff dann als Zerewitinoff-aktiven Wasserstoff (manchmal auch nur als "aktiven Wasserstoff"), wenn er nach einem von Zerewitinoff aufgefundenen Erfahren durch Umsetzung mit Methylmagnesiumjodid Methan liefert. Typische Beispiele für Verbindungen mit Zerewitinoff-aktivem Wasserstoff sind Verbindungen, die Carboxyl-, Hydroxyl-, Amino-, Imino- oder Thiol-Gruppen als funktionelle Gruppen enthalten.

Entscheidend für die Verbindungen (b) ist, daß das Grundgerüst der Moleküle der Verbindungen (b) im wesentlichen hydrophoben Charakter aufweist, d.h. im wesentlichen über einen Kohlenwasserstoffrest verfügt, der aliphatisch oder aromatisch-aliphatisch sein kann. Vorzugsweise ist das Grundgerüst der Moleküle der Verbindungen (b) aliphatisch, wobei es dann gesättigt oder ungesättigt, linear oder verzweigt sein kann.

Die Verbindungen (b) können neben der gegenüber NCO reaktiven funktionellen Gruppe, die den Zerewitinoff-aktiven Wasserstoff enthält, zusätzlich ein oder zwei weitere polare Gruppen pro Molekül enthalten. Diese polaren Gruppen können ihrerseits funktionelle Gruppen mit Zerewitinoff-aktivem Wasserstoff sein, aber auch Gruppen wie -Cl, -F oder Br.

In einer Ausführungsform weisen die hydrophoben Alkohole (b) neben ihrer gegenüber Isocyanaten reaktiven OH-Gruppe ein oder zwei weitere polare Gruppen auf, die sich gegenüber Isocyanaten inert verhalten. Erfindungsgemäß ist bevorzugt, daß als zusätzliche polare Gruppen Ether-, Ester-, Amid- und/oder Oxazolin-Gruppen vorhanden sind - unter der bereits genannten Voraussetzung, daß es sich dabei um ein oder zwei solcher Gruppen pro Molekül handelt. Beispiele hierfür sind Ricinoloxazolin und Ricinusfettsäuremethylester.

Die Verbindungen (b) enthalten vorzugsweise 6 bis 24 Kohlenstoffatome pro Molekül. Vorzugsweise enthalten diese Verbindungen als funktionelle Gruppe ausschließlich eine gegenüber NCO reaktive funktionelle Gruppe, die den Zerewitinoff-aktiven Wasserstoff, enthält. Beispiele hierfür sind Alkohole, Carbonsäuren und Amine mit 6 bis 24 Kohlenstoffatomen pro Molekül.

Vorzugsweise setzt man hydrophobe Alkohole (nachfolgend auch als Hydrophobalkohole bezeichnet), die pro Molekül zwingend eine gegenüber NCO-Gruppen reaktive OH-Gruppe enthalten müssen, als Verbindungen (b) ein. Vorzugsweise enthalten die hydrophoben Alkohole dabei insgesamt 6 bis 24 Kohlenstoffatome pro Molekül. Es ist auch möglich, daß die hydrophoben Alkohole über die zwingend pro Molekül vorhandene gegenüber NCO reaktive OH-Gruppe hinaus zusätzlich ein oder zwei OH-Gruppen enthalten. Beispiel hierfür sind alpha,beta-Diole, wie sie beispielsweise durch Umsetzung von alpha-Olefinen mit Persäuren und anschließende Ringöffnung der dabei erhaltenen Oxirane mit Wasser zugänglich sind. Besonders bevorzugt werden als Hydrophobalkohole (b) Alkohole mit 6 bis 24 C-Atomen, insbesondere 8 bis 20 C-Atomen, pro Molekül eingesetzt. Diese Alkohole können gesättigt oder ungesättigt, linear oder verzweigt sein. Fettalkohole und Oxoalkohole des genannten Kettenlängenbereichs sind besonders bevorzugt. Ganz besonders bevorzugt sind lineare gesättigte Alkohole mit 8 bis 20 C-Atomen pro Molekül, die einzeln oder als Gemisch eingesetzt werden können.

Beispiele für geeignete Alkohole (b) sind etwa:
- Streng lineare Alkohole aus natürlichen Quellen oder aus der Ziegler-Aufbaureaktion von Ethylen in Gegenwart von Aluminiumalkyl-Katalysatoren. Als besonders bevorzugte Alkohole (b) seien hier genannt: n-Octanol, n-Decanol, n-Dodecanol, n-Tetradecanol, n-Hexadecanol, n-Octadecanol.
- Mehr oder weniger stark Methyl-, Ethyl-, Propyl- Butyl- oder höher Alkyl-verzweigte Alkohole aus der Oligomerisierung und Cooligomerisierung von Olefinen, wie z.B. Propylen und/oder Butylen, ggf. zusammen mit Ethylen, mit nachfolgender edelmetallkatalysierter Hydroformylierung (wobei ggf. weitere Isomerisierungen stattfinden können). Als besonders bevorzugte Alkohole (b) seien hier genannt: Exxal 8, Exxal 9, Exxal 10, Exxal 11, Exxal 13 der Firma Exxon, i-Nonanol der Firma Degussa, sowie i-Decyl- und i-Tridecylalkohol der Firma BASF.
- Alkohole, die in 2-Stellung verzweigt sind; hierbei handelt es sich um die dem fachmann bekanten Guerbetalkohole, die durch Dimerisierung von primären Alkoholen über die sogenannte Guerbetreaktion zugänglich sind. Als besonders bevorzugte Alkohole (b) seien hier genannt: Isofol 12 der Firma Sasol, Rilanit G16 der Firma Cognis.
- Alkohole, die durch die Friedel-Crafts-Alkylierung mit oligomerisierten Olefinen erhalten werden und die dann neben einem gesättigten Kohlenwasserstoffrest einem aromatischen Ring enthalten. Als besonders bevorzugte Alkohole (b) seien hier genannt: i-Octylphenol und i-Nonylphenol.
- Als hydrophobe Alkohole (b) sind auch solche geeignet, die zwar außer der obligatorischen OH-Gruppe ein oder mehrere weitere polare Gruppen enthalten, dies aber nur in einem solchen Maße, dass der Alkohol insgesamt als hydrophobe Verbindung einzustufen ist. Als repräsentatives beispiel für derartige Alkohole sei Ricinolsäurernethylester genannt, der kommerziell unter der Bezeichnung "Edenor MeRi" von der Firma Cognis vertrieben wird.

In einer Ausführungsform können die genannten Alkohole (b) auch in Form ihrer Alkoxylierungsprodukte mit Ethylen- und/oder Propylenoxid zum Einsatz kommen.

### Zu den Komponenten (c)

Als mindestens difunktionelle Isocyanate (c) sind alle mehrfunktionellen aromatischen, alicyclischen und aliphatischen Isocyanate geeignet. Vorzugweise enthalten die geeigneten mehrfunktionellen Isocyanate im Mittel 2 bis höchstens 4 NCO- Gruppen. Diisocyanate sind als Verbindungen (c) bevorzugt.

Beispielsweise seien als geeignete Isocyanate genannt 1,5- Naphthylendiisocyanat, 4,4'-Diphenylmethandiisocyanat (MDI), hydriertes MDI (H₁₂MDI), Xylylendiisocyanat (XDI), Tetramethylxyloldiisocyanat (TMXDI), 4,4'- Diphenyldimethylmethandiisocyanat, Di- und Tetraalkyldiphenylmethandüsocyanat, 4,4'-Dibenzyldiisocyanat, 1,3-Phenylendiisocyanat, 1,4-Phenylendiisocyanat, die Isomeren des Toluylendiisocyanats (TDI), gegebenenfalls in Mischung, 1-Methyl-2,4-diisocyanato-cyclohexan, 1,6- Diisocyanato-2,2,4-trimethylhexan, 1,6-Diisocyanato-2,4,4-trimethylhexan, 1-Isocyanatomethyl-3-isocyanato-1,5,5-trimethylcyclohexan, chlorierte und bromierte Diisocyanate, phosphorhaltige Diisocyanate, 4,4'- Diisocyanatophenylperfluorethan, Tetramethoxybutan-1,4-diisocyanat, Butan- 1,4-diisocyanat, Hexan-1,6-diisocyanat (HDI), Dicyclohexylmethandiisocyanat, Cyclohexan-1,4-diisocyanat, Ethylen- diisocyanat, Phthalsäure-bis-isocyanatoethylester, ferner Polyisocyanate mit reaktionsfähigen Halogenatomen, wie 1-Chlormethylphenyl-2,4-diisocyanat, 1-Brommethylphenyl-2,6-diisocyanat, 3,3-Bis-chlormethylether-4,4'-diphenyldiisocyanat. Schwefelhaltige Polyisocyanate erhält man beispielsweise durch Umsetzung von 2 mol Hexamethylen-diisocyanat mit 1 mol Thiodiglykol oder Dihydroxydihexylsulfid. Weitere wichtige Diisocyanate sind Trimethylhexamethylendiisocyanat, 1,4-Diisocyanatobutan, 1,2-Diisocyanatododecan und Dimerfettsäure-diisocyanat. Interesse verdienen teilweise verkappte Polyisocyanate, welche die Bildung selbstvernetzender Polyurethane ermöglichen, z.B. dimeres Toluylendiisocyanat, oder mit beispielsweise Phenolen, tertiärem Butanol, Phthalimid, Caprolactam partiell umgesetzte Polyisocyanate.

Erfindungsgemäß bevorzugt ist es, dass die zur Herstellung der Polyurethane eingesetzten Isocyanate (c) zumindest überwiegend Isophorondiisocyanat (IPDI) und/oder Tetramethylxyloldiisocyanat (TMXDI) enthalten. Vorzugsweise wird Komponente (c) ausschließlich gewählt aus der Gruppe Isophorondiisocyanat (IPDI) und Tetramethylxyloldiisocyanat (TMXDI) .

In einer bevorzugten Ausführungsform werden Isocyanate mit einer Funktionalität von 2 (difunktionelle Isocyanate) eingesetzt.

In einer anderen Ausführungsform werden - anteilsweise oder ganz - Isocyanate mit einer Funktionalität oberhalb von 2 eingesetzt, wenn nämlich gewünscht ist, Polyurethane mit verzweigter Struktur herzustellen.

### Zur Herstellung der erfindungsgemäß einzusetzenden Polyurethane

Die erfindungsgemäß einzusetzenden Polyurethane können an sich nach allen dem Fachmann einschlägig bekannten Methoden hergestellt werden. Vorzugsweise weisen die Endprodukte keine freien NCO-Gruppen mehr auf bzw. sind weitgehend frei von NCO-Gruppen. Die Herstellung erfolgt vorzugsweise unter wasserfreien Bedingungen, etwa durch azeotrope Entfernung von Wasser, durch Erhitzen unter Einleiten eines Stickstoffstromes oder durch den Einsatz wasserfreier Reaktanden.

Gewünschtenfalls kann die Herstellung der Polyurethane in einem Solvens bzw. Verdünnungsmittel durchgeführt werden. Dies kann dann erforderlich sein, wenn es beim Fortschreiten der Reaktion zu einer Viskositätserhöhung kommt. Das Solvens oder Verdünnungsmittel sollte dann inert gegenüber NCO-Gruppen sein. Geeignet sind beispielsweise Benzol, Toluol, Xylol, Cyclohexan, Ethylacetat, Butylacetat sowie Dialkylether von Ethylenglykol, Diethylenglykol, und dergleichen. Solvens bzw. Verdünnungsmittel können von Anfang an im System sein oder erst im Laufe der Herstellung der Polyurethane und/oder nach deren Herstellung zugegeben werden.

Die Reaktionstemperatur ist an sich nicht kritisch. Vorzugsweise wird sie im Bereich von 40 bis 130 °C und insbesondere 60 bis 115 °C eingestellt. Dabei wählt man die Reaktionstemperatur vorzugsweise so, dass die Reaktion ausreichend schnell abläuft und Nebenprodukte minimiert werden.

In einer Ausführungsform führt man die Reaktion in Gegenwart eines Katalysators durch. Generell kommen dabei
a) metallhaltige Verbindungen, wie z.B. Dibutylzinndiacetat, Dibutylzinndidilaurat, Dibutylzinndi(2-ethyl-hexanoat), Zinnoctoat, Zinnchlorid, Kaliumoleat, Tetra(2-ethylhexyl)-titanat, Cobalt-2-ethyl-hexanoat, Eisen-2-ethyl-hexanoat, Zinknaphtenate, Eisenchlorid und/oder
b) Aminogruppen enthaltende Verbindungen, wie z.B. Triethylamin, 1,4-Diaza-bicyclooctan, 1,8-Diaza-bicyclo[5,4,0]undecen
zum Einsatz.

Das OH/ZH/NCO Verhältnis der erfindungsgemäß einzusetzenden Polyurethane, die zwingend die Bausteine (a), (b) und (c) enthalten müssen, lässt sich im Prinzip über einen weiten Bereich variieren, wobei die oben dargelegten Bedingungen einzuhalten sind.

Die Reihenfolge, in der die Bausteine (a), (b) und (c) miteinander umgesetzt werden, richtet sich generell nach den dem Fachmann allgemein bekannten Methoden.

In einer Ausführungsform werden zunächst das Polyetherpolyol (a) und das Isocyanat (c) miteinander umgesetzt. Das dabei entstehende Zwischenprodukt ist auf Grund der wie beschrieben einzuhaltenden Äquivalentverhältnisse Allophanat-haltig. Anschließend setzt man den Alkohol (c) zu.

In einer anderen Ausführungsform werden die Bausteine (a), (b) und (c) gleichzeitig eingesetzt. Dabei kann es gewünscht sein, eine innerhalb des bevorzugt genannten Bereiches relativ hohe Umsetzungstemperatur einzustellen und/oder einen Katalysator einzusetzen, der die Bildung von intermediären Allophanatstrukturen begünstigt.

In allen Fällen ist es bevorzugt, die Reaktion so weit zu führen, dass das Produkt einen nur minimalen Rest-NCO-Gehalt aufweist. Vorzugsweise wird dieser Rest-NCO-Gehalt auf einen Wert von null eingestellt, d.h. man verfolgt den Verlauf der Reaktion durch Probenahme und Bestimmung des NCO-Gehaltes und bricht die Reaktion ab, sobald kein Rest-NCO-Gehalt mehr nachweisbar ist.

### Verdickerkonzentrate

Ein weiterer Erfindungsgegenstand sind Verdickerkonzentrate enthaltend
(A) Wasser,
(B) nicht-ionische, in Wasser dispergierbare oder in Wasser lösliche Polyurethane gemäß einem der Ansprüche 1-4 und
(C) gegebenenfalls ein oder mehrere organische Lösungsmittel und/oder nichtionische Tenside vom Typ der Anlagerungsverbindungen von Ethylen- und/oder Propylenoxid an Alkohole mit 8-18 C-Atomen.

Bezüglich bevorzugter Ausführungsformen im Hinblick auf die Verbindungen (B) gilt das oben Ausgeführte.

Bei den Lösungsmitteln (C) handelt es sich um flüchtige organische Lösungsmittel. Beispiele hiefür sind niedermolekulare Alkohole wie Methanol, Ethanol, n-Propanol, i-Propanol, n-Butanol, i-Butanol, sec-Butanol, Ethandiol, Propandiol, Butandiol, Glycerin, Trimethylolpropan in Betracht.

Als nichtionische Tenside vom Typ der Anlagerungsverbindungen von Ethylen- und/oder Propylenoxid an Alkohole mit 8-18 C-Atomen (C) sind solche mit 2 bis 4 Mol Ethylenoxid pro mol Alkohol bevorzugt. Das Kohlenstoffgerüst der Alkohole kann dabei gesättigt oder ungesättigt, linear oder verzweigt sein. Als Beispiel einer geeigneten Verbindung (C) dieser Klasse sei etwa Dehydol 04 (Handelsprodukt der Firma Cognis Deutschland GmbH & Co. KG) genannt, ein Anlagerungsprodukt von 4 mol Ethylenoxid pro mol Octanol.

Desweiteren betrifft die Erfindung die Verwendung der erfindungsgemäßen Verdickungsmittel bzw. Verdickerkonzentrate zur Verdickung wäßriger Systeme, bevorzugt wäßriger Dispersionen, ausgewählt aus der Gruppe bestehend aus wäßrigen Kraftfahrzeug- und Industrielacken, Druck- und Textilfarben, Pigmentdruckpasten, wäßrigen pharmazeutischen Formulierungen, kosmetischen Formulierungen oder pharmazeutisch-kosmetischen Formulierungen, Pflanzenschutzformulierungen, Füllstoff- und Pigmentdispersionen, Putz- und Anstrichmittel, Zubereitungen von Waschmitteln, Klebstoffen, Wachsen und Polituren sowie für die Erdölförderung.

### Beispiele

### Verwendete Abkürzungen

| | |
|---|---|
| PEG: | Polyglycol E 8000 (Polyethylenglykol der Fa. Dow Chemical; Hydroxylzahl =13) |
| IPDI: | Isophorondiisocyanat (IPDI, Fa. Degussa/Hüls) |
| TMXDI: | m-Tetramethylxyloldiisocyanat (TMXDI, Fa. Cytec) |
| i-C₁₀-OH: | Isodecylalkohol (oxomethyliertes Propentrimer; Handelsprodukt "Exxal 10" der Firma Exxon Mobil Chemical) |
| BuOc-OH: | 2-Butyl-1-octanol |
| Dehydol 04 | Anlagerungsprodukt von 4 mol Ethylenoxid an 1 mol n-Octanol (Fa. Cognis) |

### Polyurethanherstellung

### Beispiel 1 (zum Vergleich)

Im 11-Vierhalskolben wurden 207,1g (24 mmol) Polyglycol E 8000 (Polyethylenglykol der Dow Chemical; OHZ=13) vorgelegt. Es wurde zweimal evakuiert und mit Stickstoff belüftet. Anschließend wurde Vakuum angelegt und der Ansatz auf 110°C erhitzt. Bei dieser Temperatur wurde bei einem Vakuum von mindestens 10 mbar über einen Zeitraum von zwei Stunden entwässert. Dann wurde mit Stickstoff belüftet und im weiteren Verlauf die Schutzgasatmosphäre durch eine leichten Stickstoffstrom aufrechterhalten. Über die gesamte folgende Reaktionszeit wurde mit einer Rührerdrehzahl von 120 Upm gerührt. Danach wurden nacheinander 7,6 g (48 mmol) Isodecylalkohol und 10,7g (48 mmol) Isophorondiisocyanat (IPDI, Fa. Degussa/Hüls) zugegeben. Die Reaktionstemperatur wurde während der Zugabe und während der folgenden Reaktionszeit bei 110°C gehalten.

Nach einer Stunde Reaktionszeit wurde (als Katalysator) 0,05g 1,8-Diazabicyclo[5-4-0]undecen-7 (Fa. Nitroil) zugegeben.

Sobald kein Restisocyanat mehr nachweisbar war (dies war nach ca. zwei Stunden Gesamtreaktionszeit der Fall), wurden ohne weitere Heiz- oder Kühlmassnahmen zunächst 143,6 g Dehydol 04 deo (Anlagerungsprodukt von 4 Mol Ethylenoxid an 1 Mol n-Octanol; Handelsprodukt der Fa. Cognis) zugesetzt und bis zur Homogenität gerührt.

Dabei sank die Temperatur auf unter 100 °C ab. Anschließend wurden unter Rühren 359,0 g deionisiertes Wasser zugesetzt und bis zum Erreichen der Homogenität gerührt.

Es wurden ca. 700 g einer viskosen, klaren, leicht gelblichen Polymerlösung aus dem Reaktionsgefäß isoliert.

Der Trockenrückstand (zu dessen Bestimmung wurden ca. 1-2 g der wie beschrieben hergestellten Polymerlösung in einem 10-cm-Aluschälchen bei 105°C über 1,5 h im Umlufttrockenschrank getrocknet) betrug 48,6 Gew.-% und die Brookfield-Viskosität 2,75 Pas (Brookfield RVT-Viskosimeter mit Spindel 6 bei 20 Umdrehungen pro Minute und 22°C).

### Beispiel 1a (erfindungsgemäß)

Es wurde analog zu Beispiel 1 verfahren, wobei jedoch anstelle der in Beispiel 1 eingesetzten 7,6 g (48 mmol) 11,4 g (72 mmol) Isodecylakohol und anstelle der in Beispiel 1 eingesetzten 10,7 g (48 mmol) 15,1g (68 mmol) Isophorondiisocyanat eingesetzt wurden. Außerdem kamen 146,9g statt 143,6 g Dehydol 04 deo und 367,3 g statt 359,0 g deionisiertes Wasser zum Einsatz.

Es wurden ca. 730 g einer viskosen, klaren, leicht gelblichen Polymerlösung aus dem Reaktionsgefäß isoliert.

Der Trockenrückstand betrug 48,4 Gew.-%.

Die Viskosität der erhaltenen Polymerlösung betrug 4,75 Pas.

### Beispiel 1b (erfindungsgemäß)

Es wurde analog zu Beispiel 1 verfahren, wobei jedoch anstelle der in Beispiel 1 eingesetzten 7,6 g (48 mmol) 13,3 g (84 mmol) Isodecylakohol und anstelle der in Beispiel 1 eingesetzten 10,7 g (48 mmol) 16,5 g (74 mmol) Isophorondiisocyanat eingesetzt wurden. Außerdem kamen 149,5 g statt 143,6 g Dehydol 04 deo und 373,8 g statt 359,0 g deionisiertes Wasser zum Einsatz.

Restisocyanat war erst nach drei Stunden Reaktionszeit nicht mehr nachweisbar.

Es wurden ca. 740 g einer viskosen, klaren, leicht gelblichen Polymerlösung aus dem Reaktionsgefäß isoliert.

Der Trockenrückstand betrug 49,1 Gew.-%.

Die Viskosität der erhaltenen Polymerlösung betrug 4,0 Pas.

### Beispiel 1c (erfindungsgemäß)

Es wurde analog zu Beispiel 1 verfahren, wobei jedoch anstelle der in Beispiel 1 eingesetzten 7,6 g (48 mmol) 15,2 g (96 mmol) Isodecylakohol und anstelle der in Beispiel 1 eingesetzten 10,7 g (48 mmol) 18,1g (82 mmol) Isophorondiisocyanat eingesetzt wurden. Außerdem kamen 150,8g statt 143,6 g Dehydol 04 deo und 377,0 g statt 359,0 g deionisiertes Wasser zum Einsatz.

Es wurden ca. 750 g einer viskosen, klaren, leicht gelblichen Polymerlösung aus dem Reaktionsgefäß isoliert.

Der Trockenrückstand betrug 49,2 Gew.-%.

Die Viskosität der erhaltenen Polymerlösung betrug 5 Pas.

### Beispiel 2 (zum Vergleich)

Im 11-Vierhalskolben wurden 207,1g (24 mmol) Polyglycol E 8000 (Polyethylenglykol der Dow Chemical; OHZ=13) vorgelegt. Es wurde zweimal evakuiert und mit Stickstoff belüftet. Anschließend wurde Vakuum angelegt und der Ansatz auf 110°C erhitzt. Bei dieser Temperatur wurde bei einem Vakuum von mindestens 10 mbar über einen Zeitraum von zwei Stunden entwässert. Dann wurde mit Stickstoff belüftet und im weiteren Verlauf die Schutzgasatmosphäre durch eine leichten Stickstoffstrom aufrechterhalten. Über die gesamte folgende Reaktionszeit wurde mit einer Rührerdrehzahl von 120 Upm gerührt. Danach wurden nacheinander 8,9 g (48 mmol) 2-Butyl-1-octanol, 11,7g (48 mmol) m-Tetramethylxyloldiisocyanat (TMXDI, Fa. Cytec) und dann (als Katalysator) 0,05g 1,8-Diazabicyclo[5-4-0]undecen-7 (Fa. Nitroil) zugegeben. Die Reaktionstemperatur wurde der Zugabe und während der folgenden Reaktionszeit bei 110°C gehalten.

Sobald kein Restisocyanat mehr nachweisbar war (dies war nach ca. sechs Stunden der Fall), wurden ohne weitere Heiz- oder Kühlmassnahmen zunächst 139,1 g Dehydol 04 deo (Anlagerungsprodukt von 4 Mol Ethylenoxid an 1 Mol n-Octanol; Handelsprodukt der Fa. Cognis) zugesetzt und bis zur Homogenität gerührt. Dabei sank die Temperatur auf unter 100 °C ab. Anschließend wurden unter Rühren 347,8 g deionisiertes Wasser zugesetzt und bis zum Erreichen der Homogenität gerührt.

Es wurden ca. 700 g einer viskosen, klaren, leicht gelblichen Polymerlösung aus dem Reaktionsgefäß isoliert.

Der Trockenrückstand (zu dessen Bestimmung wurden ca. 1-2 g der wie beschrieben hergestellten Polymerlösung in einem 10-cm-Aluschälchen bei 105°C über 1,5 h im Umlufttrockenschrank getrocknet) betrug 48,0 Gew.-% und die Brookfield-Viskosität 3,25 Pas (Brookfield RVT-Viskosimeter mit Spindel 6 bei 20 Umdrehungen pro Minute und 22°C).

### Beispiel 2a (erfindungsgemäß)

Analog der Vorgehensweise in Beispiel 2 wurden folgende Einsatzmengen verarbeitet:
186,3 g (22 mmol) Polyglycol E 8000 (Polyethylenglykol der Dow Chemical; OHZ=13)
9,9 g (53 mmol) 2-Butyl-1-octanol
12,9 g (53 mmol) m-Tetramethylxyloldiisocyanat
0,05g 1,8-Diazabicyclo[5-4-0]undecen-7 (Fa. Nitroil)
127,1 g Dehydol 04 deo
317,7 g deionisiertes Wasser

Es wurden ca. 640 g einer viskosen, klaren, leicht gelblichen Polymerlösung aus dem Reaktionsgefäß isoliert.

Der Trockenrückstand betrug 48,6 Gew.-%.

Die Viskosität der erhaltenen Polymerlösung betrug 3,25 Pas.

### Beispiel 2b (erfindungsgemäß)

Analog der Vorgehensweise in Beispiel 2 wurden folgende Einsatzmengen verarbeitet:
165,7g (19Mol) Polyglycol E 8000 (Polyethylenglykol der Dow Chemical; OHZ=13)
10,8 (58 mmol) 2-Butyl-1-octanol
12,9 g (53 mmol) m-Tetramethylxyloldiisocyanat
0,05g 1,8-Diazabicyclo[5-4-0]undecen-7 (Fa. Nitroil)
114,1 g Dehydol 04 deo
285,2 g deionisiertes Wasser

Es wurden ca. 570 g einer viskosen, klaren, leicht gelblichen Polymerlösung aus dem Reaktionsgefäß isoliert.

Der Trockenrückstand betrug 48,9 Gew.-%.

Die Viskosität der erhaltenen Polymerlösung betrug 3,0 Pas.

### Beispiel 2c (erfindungsgemäß)

Analog der Vorgehensweise in Beispiel 2 wurden folgende Einsatzmengen verarbeitet:
207,1g (24mol) Polyglycol E 8000 (Polyethylenglykol der Dow Chemikal; OHZ=13)
13,6 (73 mmol) 2-Butyl-1-octanol
17,2 g (71 mmol) m-Tetramethylxyloldiisocyanat
0,05g 1,8-Diazabieyclo[5-4-0]undecen-7 (Fa. Nitroil)
143,7 g Dehydol 04 deo
359,2 g deionisiertes Wasser

Es wurden ca. 720 g einer viskosen, klaren, leicht gelblichen Polymerlösung aus dem Reaktionsgefäß isoliert.

Der Trockenrückstand betrug 48,3 Gew.-%.

Die Viskosität der erhaltenen Polymerlösung betrug 3,75 Pas.

### Beispiel 2d (erfindungsgemäß)

Analog der Vorgehensweise in Beispiel 2 wurden folgende Einsatzmengen verarbeitet:
434,9g (50mol) Polyglycol E 8000 (Polyethylenglykol der Dow Chemical; OHZ=13) 37,6 (202 mmol) 2-Butyl-1-octanol
39,5 g (162 mmol) m-Tetramethylxyloldiisocyanat
0,11 g 1,8-Diazabicyclo[5-4-0]undecen-7 (Fa. Nitroil)
330,3 g Dehydol 04 deo
825,8 g deionisiertes Wasser

In diesem Fall war Restisocyanat schon nach zwei Stunden Reaktionszeit nicht mehr nachweisbar.

Es wurden ca. 1650 g einer viskosen, klaren, leicht gelblichen Polymerlösung aus dem Reaktionsgefäß isoliert.

Der Trockemückstand betrug 48,2 Gew.-%.

Die Viskosität der erhaltenen Polymerlösung betrug 2,25 Pas.

### Bestimmung der Dispersionsverdickung

0,2% des jeweiligen polymeren Wirkstoffs gemäß den obigen Beispiel (bezogen und berechnet auf Aktivstubstanz ohne Dehydol 04) wurden mit 0,41g einer Mischung aus 31,4 Gew.-% Propandiol und 68,6 Gew.-% Wasser homogenisiert.

Danach wurden 20g der wäßrigen Polyacrylatdispersion Neocryl XK 90 (45 % Feststoffgehalt; Neo Resins) zugesetzt und die Mischung mit einem Holzspatel ca. zwei Minuten homogen gerührt.

Nach einer Standzeit von 20 Stunden wurde erneut mit einem Holzspatel vorsichtig durchgerührt.

Jetzt wurde mit einem Brookfield Kegel-Platte-Viskosimeter Haake RC20-CPS-P mit Kegel C50-1 die Viskosität bei einer Scherrate von 300 s⁻¹ und bei einer Scherrate von 4800 s⁻¹ gemessen.

Mit der gleichen Probe wurde an einem Epprecht Kegel-Platte-Viskosimeter mit dem Messkegel C die ICI-Viskosität bei 10000 sec⁻¹ gemessen.

In gleicher Weise wurde auf dem Brookfield Kegel-Platte-Viskosimeter Haake RC20-CPS-P mit Kegel C50-1 die Verdickungswirkung des Polyurethans in einer wäßrigen Vinylacetat-Ethylen-Copolymerdispersion Mowilith LDM 1871 (53 % Feststoffgehalt; Clariant) bei einer Scherrate von 300 s⁻¹ und bei einer Scherrate von 4800 s⁻¹ bestimmt.

Die erhaltenen Viskositätswerte sind in den beiden folgenden Tabellen 1 und 2 zusammengestellt. Die erste Spalte der Tabelle zeigt jeweils, welcher polymere Wirkstoff als HEUR-Verdicker eingesetzt wurde, die zweite bis fünfte Spalte der Tabellen nennt der Übersicht halber jeweils die dem Verdicker zugeordneten Strukturparameter (deren Werte bereits den obigen Beispielen entnommen werden können).

**Tabelle 1**

| | | | | | **Dispersionsviskosität C/P [mPas]** | | | | | |
|---|---|---|---|---|---|---|---|---|---|---|
| | | | | | **Neocryl XK90+Verdünner** | | | **Mowilith LDM 1871+Verdünner** | | |
| **Polymer gemäß Beispiel** | **Verhältnis PEG : i-C₁₀-OH :** IPDI [molar] | **Verhältnis PEG : i-C₁₀-OH :** IPDI [equivalent] | **x** | **y** | 300s | 4800s⁻¹ | **ICI** Cone C, 25°C/ 10000 | 300s⁻¹ | 4800s⁻¹ | **ICI** Cone C 25°C/ 10000 |
| 1 | 1:2:2 | 1:1:2 | 0 | 0 | 665 | 90 | 80 | 250 | 65 | 40 |
| 1a | 1:3:2,8 | 1:1,5 :2,8 | 0,5 | 0,4 | 1590 | 135 | 90 | 480 | 85 | 60 |
| 1b | 1:3,5:3,1 | 1:1,75 : 3,1 | 0,75 | 0,55 | 1420 | 120 | 90 | 420 | 80 | 70 |
| 1c | 1:4:3,4 | 1:2:3,4 | 1 | 0,7 | 1800 | 135 | 90 | 555 | 90 | 70 |

**Tabelle 2**

| | | | | | **Dispersionsviskosität C/P** | | | | | |
|---|---|---|---|---|---|---|---|---|---|---|
| | | | | | **Neocryl XK90+Thinner** | | | **Mowilith LDM 1871+Thinner** | | |
| **Polymer gemäß Beispiel** | **Verhältnis PEG : BuOc-OH : TMXDI** [molar] | **Verhältnis PEG : BuOc-OH : TMXDI** [equivalent] | **x** | **y** | 300s⁻¹ | 4800s⁻¹ | **ICI** Cone C, 25°C/ 10000 | 300s⁻¹ | 4800s⁻¹ | **ICI** Cone C 25°C/ 10000 |
| 2 | 1 : 2 : 2 | 1 : 1 : 2 | 0 | 0 | 680 | 145 | 90 | 250 | 100 | 120 |
| 2a | 1 : 2 ,4 : 2,4 | 1 : 1,2 : 2,4 | 0,2 | 0,2 | 980 | 170 | 100 | 330 | 110 | 130 |
| 2b | 1 : 3 : 2,8 | 1 : 1,5 : 2,8 | 0,5 | 0,4 | 1200 | 185 | 110 | 330 | 100 | 150 |
| 2c | 1 : 3 : 3 | 1 : 1,5 : 3 | 0,5 | 0,5 | 1280 | 185 | 120 | 480 | 120 | 140 |
| 2d | 1 : 4 . 3,2 | 1 : 2 : 3,2 | 1 | 0,6 | 1350 | 190 | 110 | 450 | 110 | 140 |

### Fazit

Es zeigt sich, dass mit dem erfindungsgemäßen Polyurethanen insgesamt eine deutlich verbesserte Verdickungswirksamkeit erreicht wird.

## Patentansprüche

1. Verdickungsmittel, die auf einer wäßrigen Zubereitung nicht-ionischer, in Wasser dispergierbarer oder in Wasser löslicher Polyurethane basieren, wobei diese Polyurethane herstellbar sind durch Umsetzung von
(a) ein oder mehreren hydrophilen Polyolen, die pro Molekül mindestens zwei OH-Gruppen und mindestens zwei funktionelle Gruppen, die ausgewählt sind aus den Funktionen - O- (Ethergruppen) und -COO- (Estergruppen), wobei das Molgewicht dieser hydrophilen Verbindungen mindestens 300 beträgt, enthalten,
(b) ein oder mehreren hydrophoben Verbindungen mit mindestens einem Zerewitinoff-aktiven Wasserstoffatom pro Molekül, wobei das Molgewicht dieser hydrophoben Verbindungen im Bereich von 100 bis 500 liegt und wobei pro Molekül dieser hydrophoben Verbindungen mindestens eine lineare oder verzweigte, gesättigte oder ungesättigte Alkylkette mit mindestens fünf aufeinander folgenden C-Atomen enthalten ist, die nicht mit Heteroatomen verknüpft ist, und
(c) ein oder mehreren, mindestens difunktionellen, Isocyanaten,
**dadurch gekennzeichnet, dass** die Verbindungen a), b) und c) in den Äqivalentverhältnissen OHₐ₎ : ZH_{b)} : NCO_{c)} von 1 : (1 + x) : 2(1 + y) miteinander umgesetzt werden, mit den Maßgaben, daß folgende Bedingungen gelten:
• x ist eine Zahl im Bereich von 0,05 bis 1,2 ,
• y ist eine Zahl im Bereich von (0,2 bis 1,05) x und
• Äquivalentverhältnis NCO_{c)} > (OHₐ₎ + ZH_{b)}).

2. Verdickungsmittel gemäß Anspruch 1, wobei Komponente (a) gewählt wird aus der Gruppe der Polyethylenglykole mit einem Molgewicht im Bereich von 1.000 bis 15.000.

3. Verdickungsmittel gemäß Anspruch 1 oder 2, wobei Komponente (b) gewählt wird aus der Gruppe der Fettalkohole mit 6 bis 24 C-Atomen.

4. Verdickungsmittel gemäß einem der Ansprüche 1 bis 3, wobei Komponente (c) gewählt wird aus der Gruppe Isophorondiisocyanat und Tetramethylxyloldiisocyanat.

5. Verdickerkonzentrate enthaltend
(A) Wasser,
(B) nicht-ionische, in Wasser dispergierbare oder in Wasser lösliche Polyurethane gemäß einem der Ansprüche 1 bis 4 und
(C) gegebenenfalls ein oder mehrere organische Lösungsmittel und/oder nicht-ionische Tenside vom Typ der Anlagerungsverbindungen von Ethylen- und/oder Propylenoxid an Alkohole mit 8-18 C-Atomen.

6. Verwendung der Verdickungsmittel gemäß den Ansprüchen 1 bis 4 zur Verdickung von Dispersionsfarben.

7. Verwendung der Verdickungsmittel gemäß den Ansprüchen 1 bis 4 zur Verdickung von wäßrigen Dispersionen.

8. Verwendung nach Anspruch 7, wobei es sich bei den wässrigen Dispersionen um kosmetische Zubereitungen handelt.

9. Verwendung nach Anspruch 7, wobei es sich bei den wässrigen Dispersionen um Putz- und Anstrichmittel handelt.

## Claims

1. Thickeners based on an aqueous preparation of nonionic, water-dispersible or water-soluble polyurethanes, these polyurethanes being obtainable by reaction of
(a) one or more hydrophilic polyols (a) which contain per molecule at least two OH groups and at least two functional groups selected from the functions -O- (ether groups) and -COO- (ester groups), the molecular weight of these hydrophilic compounds being at least 300,
(b) one or more hydrophobic compounds containing at least one zerewitinoff-active hydrogen atom per molecule, the molecular weight of these hydrophobic compounds being in the range from 100 to 500 and at least one linear or branched, saturated or unsaturated alkyl chain with at least five consecutive carbon atoms, which is not linked to hetero atoms, being present per molecule of these hydrophobic compounds, and
(c) one or more at least difunctional isocyanates,
**characterized in that** the compounds a), b) and c) are reacted with one another in equivalent ratios of OHₐ₎:ZH_{b)}:NCO_{c)} of 1 : (1+x) : 2(1+y), with the following provisos:
• x is a number of 0.05 to 1.2 and
• y is a number of (0.2 to 1.05)x.

2. Thickeners as claimed in claim 1, **characterized in that** component (a) is selected from the group of polyethylene glycols having a molar weight in the range from 1,000 to 15,000.

3. Thickeners as claimed in claim 1 or 2, **characterized in that** component (b) is selected from the group of C₆₋₂₄ fatty alcohols.

4. Thickeners as claimed in any of claims 1 to 3, **characterized in that** component (c) is selected from the group consisting of isophorone diisocyanate and tetramethyl xylene diisocyanate.

5. Thickener concentrates containing
(A) water,
(B) nonionic, water-dispersible or water-soluble polyurethanes according to any of claims 1 to 4 and
(C) optionally one or more organic solvents and/or nonionic surfactants in the form of addition compounds of ethylene and/or propylene oxide onto C₈₋₁₈ alcohols.

6. Use of the thickeners claimed in claims 1 to 4 for thickening emulsion paints.

7. Use of the thickeners claimed in claims 1 to 4 for thickening aqueous dispersions.

8. Use claimed in claim 7, **characterized in that** the aqueous dispersions are cosmetic preparations.

9. Use claimed in claim 7, **characterized in that** the aqueous dispersions are cleaning and coating compositions.

## Revendications

1. Épaississants, qui sont à base d'une préparation aqueuse de polyuréthannes non ioniques, solubles dans l'eau ou dispersables dans l'eau, ces polyuréthannes pouvant être préparés par mise en réaction de
(a) un ou plusieurs polyols hydrophiles qui contiennent par molécule au moins deux groupes OH et au moins deux groupes fonctionnels qui sont choisis parmi les fonctions -O- (groupes éther) et -COO- (groupes ester), la masse moléculaire de ces composés hydrophiles étant d'au moins 300,
(b) un ou plusieurs composés hydrophobes comportant au moins un atome d'hydrogène actif dans la réaction de Zerewetinoff par molécule, la masse moléculaire de ces composés hydrophobes se situant dans la plage de 100 à 500 et, par molécule de ces composés hydrophobes, étant contenue au moins une chaîne alkyle linéaire ou ramifiée, saturée ou insaturée, comportant au moins cinq atomes de carbone successifs, qui n'est pas liée à des hétéroatomes, et
(c) un ou plusieurs isocyanates au moins difonctionnels,
**caractérisés en ce qu'**on fait réagir entre eux les composés a), b) et c) en les rapports d'équivalents OHₐ₎ : ZH_{b)} : NCO_{c)} de 1 : (1 + x) : 2(1 + y), avec les conditions que les conditions suivantes soient remplies :
• x est un nombre dans la plage de 0,05 à 1,2,
• y est un nombre dans la plage de (0,2 à 1,05)x et
• le rapport d'équivalents NCO_{c)} > (OHₐ₎ + ZH_{b)}).

2. Épaississants selon la revendication 1, dans lesquels le composant (a) est choisi dans le groupe des polyéthylèneglycols ayant une masse moléculaire dans la plage de 1 000 à 15 000.

3. Épaississants selon la revendication 1 ou 2, dans lesquels le composant (b) est choisi dans le groupe des alcools gras ayant de 6 à 24 atomes de carbone.

4. Épaississants selon l'une quelconque des revendications 1 à 3, dans lesquels le composant (c) est choisi dans le groupe constitué par l'isophorone-diisocyanate et le tétraméthylxylène-diisocyanate.

5. Concentrés d'épaississant, contenant
(A) de l'eau,
(B) des polyuréthannes non ioniques, solubles dans l'eau ou dispersables dans l'eau, selon l'une quelconque des revendications 1 à 4 et
(C) éventuellement un ou plusieurs solvants organiques et/ou tensioactifs non ioniques du type des composés de fixation par addition d'oxyde d'éthylène et/ou d'oxyde de propylène sur des alcools ayant de 1 à 18 atomes de carbone.

6. Utilisation des épaississants selon les revendications 1 à 4, pour l'épaississement de peintures en dispersion.

7. Utilisation des épaississants selon les revendications 1 à 4, pour l'épaississement de dispersions aqueuses.

8. Utilisation selon la revendication 7, dans laquelle les dispersions aqueuses consistent en des préparations cosmétiques.

9. Utilisation selon la revendication 7, dans laquelle les dispersions aqueuses consistent en des enduits et des peintures.
